# EUROPEAN PATENT SPECIFICATION

(11) **EP 3 338 817 B1**
(45) Date of publication and mention of the grant of the patent: **12.02.2020**
(21) Application number: 17210150.3
(22) Date of filing: 22.12.2017
(51) Int. Cl.: A61L 27/18, A61L 27/20

(54) **PROSTHESIS WITH A CHITOSAN CORE FOR REGENERATION OF NERVES AND METHOD OF ITS MANUFACTURING**
PROTHESE MIT EINEM CHITOSANKERN FÜR ZUR REGENERATION DER NERVEN UND VERFAHREN ZU DEREN HERSTELLUNG
PROTHÈSE COMPRENANT UN NOYAU DE CHITOSANE POUR LA RÉGÉNÉRATION DE NERFS ET SON PROCÉDÉ DE FABRICATION

(30) Priority: 22.12.2016 PL 41994316
(43) Date of publication of application: 27.06.2018
(73) Proprietor: Slaski Uniwersytet Medyczny w Katowicach, 40-055 Katowice (PL); Instytut Biopolimerów I Wlókien Chemicznych, 90-570 Lódz (PL)
(72) Inventor: MARCOL, Wieslaw, 44-348 Skrzyszów (PL); WLASZCZUK, Adam, 43-190 Mikolów (PL); LEWIN-KOWALIK, Joanna, 40-771 Katowice (PL); WAWRO, Dariusz, 91-404 Lódz (PL); KUCHARSKA, Magdalena, 94-056 Lódz (PL)
(74) Representative: Augustyniak, Magdalena Anna

(56) References cited:
- EP-A1- 2 228 036
- WO-A1-2010/062297
- PL-B1- 218 618

## Description

The invention discloses a prosthesis for regeneration of nerves which consists of a core made from chitosan placed in a sleeve in the shape of a cylinder, the said sleeve being made from DL-lactide/glycolide copolymer and a method of manufacturing such a prosthesis.

Nerve injuries are a consequence of accidents, tumour resection or adverse effects of surgeries. These injuries result in a loss of motor skills and atrophy of denervated muscles. Nerve injuries are frequently associated with neuropathic pain refractory to pharmacological treatment. In the absence of any protection, nerve fibres located in the proximal segment of the severed nerve spontaneously and chaotically re-grow and form neuromas or microneuromas. Therefore, it is necessary to protect the formed nerve stump, wherein a coupling that restores the original nerve pathway would provide an ideal solution.

Three methods are used for the surgical treatment of damaged nerves: a direct coupling of ends of the severed nerve, an autograft and a use of prostheses from synthetic or natural materials for regeneration of nerves.

The first reconstruction method may be used only for losses shorter than 1 cm, wherein a reconstruction and a healing should occur without any tension of the nerve and its surrounding tissues. Autografts are used with longer losses. In this case, so called "bridging" with a nerve fragment taken from a different site of the body is used to reconstruct the fragment lost. This method, however, results in the additional mutilation of the patient and, therefore, alternative treatment modalities are currently being developed, such as those based on prostheses for regeneration of nerves. Such prostheses are typically prepared from biomaterials resorbable in the organism, such as proteins and polysaccharides as well as lactic acid copolymers in a form of fibres, films and sponges.

The publication Preparation of chitosan nanofiber tube by electrospinning. J Nanosci Nanotechnol. 2007 Mar; 7(3):852-5 discloses nerve prostheses made from chitosan nanofibres coated with a chitosan layer. The chitosan nanofibres were found to be superior substrate than a chitosan film for growing cells and they can be used for the manufacture of tubes that support regeneration of nerves.

The publication Enhanced nerve regeneration through a bilayered chitosan tube: the effect of introduction of glycine spacer into the CYIGSR sequence. J Biomed Mater Res A. 2008 Jun 15;85(4):919-28 discloses prostheses of nerves prepared in the form of a bilayer tube which consists of a chitosan film (on the outside) and chitosan nanofibres (inside). Laminin peptides elongated with a fragment consisting of multiple glycine molecules were connected with a nanofibre layer. When comparing the prostheses consisted of nanofibres solely with a deacetylation degree of 78 and 93%, from a chitosan film with a deacetylation degree of 93% as well as bilayer prostheses (nanofibres with a deacetylation degree of 78% and 93% and film: 93%), the investigators found that even though the recovery of motor functions was delayed in all groups, sensory functions recovered first in the rats implanted with a prosthesis from nanofibres with a deacetylation degree of 93%, immediately after the group in which autologous grafts were used.

The publications Brain Res., Vol. 128, 2005, p. 1897-1910 and Microsur., 2008, p. 238-242 and p. 471-479 disclose prostheses in the form of tube made from chitosan and polyglycolic acid.

Patent US 7.135.040 discloses prostheses for the reconstruction of peripheral nerves in the form of woven biodegradable tubes from L-lactic acid and glycolide copolymer (10:90) and chitosan.

Patent US 6.090.117 discloses temporary nerve junctions in the form of tubes made from lactic acid copolymers filled with a collagen gel with addition of laminin and a growth factor, in which in turn collagen fibres are additionally immersed.

Patent PL 218618 by IBWCh/SUM discloses prostheses for the reconstruction of peripheral nerves which consist of a cylindrical core made from microcrystalline chitosan with 7 to 13 pass-through channels that are parallel to the core axis. The core is placed in a sleeve made from DL-lactide/glycolide copolymer or from microcrystalline chitosan.

In spite of an intensive research into the development of prostheses that support regeneration of nerves, it is still problematic to ensure an optimum path through the prosthetic structure for the regenerating nerve fibres and thus it is problematic to achieve a good effectiveness of regeneration of nerves.

Accordingly, the object of this invention was to develop a prosthesis for regeneration of nerves that would enable a rapid and an effective coupling of nerve stumps while ensuring a good regeneration effectiveness with a simultaneous limited formation of microneuromas that result in a neuropathic pain.

The prosthesis for regeneration of nerves of the invention consists of a core with a length of 5-50 mm placed in a sleeve in the shape of a cylinder with a wall thickness of 0.04-1.0 mm and an internal diameter of 1-10 mm. The sleeve is made from DL-lactide/glycolide copolymer with a molar ratio of 75/25, and the core is made from chitosan with a mean molecular weight of 100-500 kDa and a deacetylation degree of 80-98%, wherein the chitosan is in the form of solid fibres with a diameter of 20-40 µm. The chitosan fibres, that constitute the core are placed in the sleeve in parallel to the axis of the said sleeve and they are present in a strained state, with their quantity being from 150-22500.

In addition, the core is placed in the sleeve so that the sleeve protrudes beyond both core ends.

The method of manufacturing the prosthesis for regeneration of nerves comprising the following steps:
a) forming a film from DL-lactide/glycolide copolymer by:
   - pouring a solution of DL-lactide/glycolide copolymer with a molar ratio of 75/25 in 1,4-dioxane with a concentration of 3% by weight on a Teflon mould;
   - leaving the poured solution at room temperature for 24 hours and subsequently drying it at 50°C at 0.07 MPa for 4 days, whereby the film is formed;
   - rinsing the formed film several times with demineralised water with glycerine;
   - freeze-drying the film for 24 hours at -25°C at 0.1 millibar;
b) forming a sleeve in the shape of a cylinder with a wall thickness of 0.04-1.0 mm and an internal diameter of 1-10 mm from the film obtained in the step a);
c) forming a prosthesis core by placing into the sleeve obtained in the step b) between 150 and 22500 solid chitosan fibres with a mean molecular weight of 100-500 kDa, a deacetylation degree of 80-98% and a diameter of 20-40 µm, wherein the fibres are placed in a strained state and in parallel to the sleeve axis, and the formed core has a length of 5-50 mm and the sleeve protrudes beyond both core ends.

The chitosan fibres used as the core in the prostheses of the invention act as a scaffold over which the regenerating nerve fibres normally re-grow towards the distal stump. The large number of chitosan fibres constituting the core and the large volume of free space therebetween are of prime importance. Owing to this solution, the nerve fibres may freely select the most convenient path through the structure of the prosthesis core until joining the distal stump. The size of the space available to the re-growing nerve fibres has a favourable effect on the effectiveness of the regeneration as was confirmed by the performed tests.

An advantage of the invention is also the use of materials for the manufacture of prostheses which are biocompatible and resorbable in the organism.

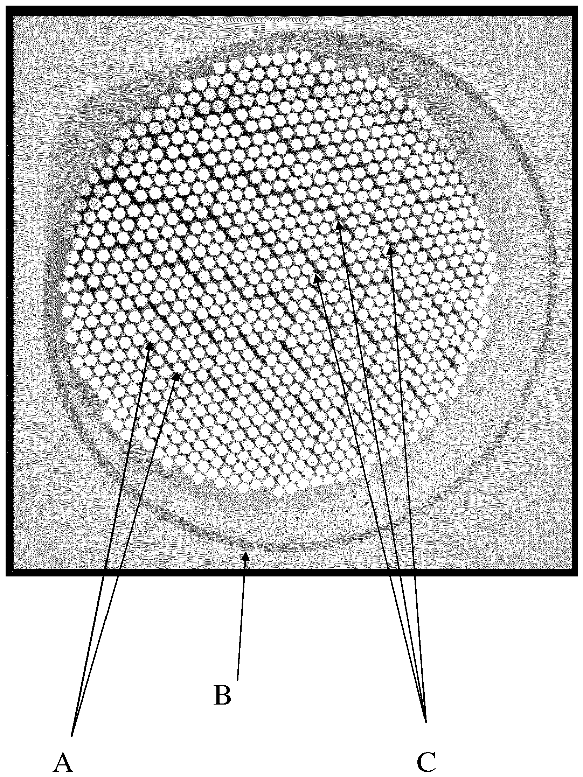

The prosthesis of the invention is illustrated schematically in the Figure which shows a perspective view of the prosthesis.

The invention is illustrated by the following examples.

### Example I

The prosthesis for regeneration of nerves illustrated schematically in the Figure consists of a core with a length of 7 mm placed in a sleeve (B) in the shape of a cylinder with a thickness of 0.045 mm and an internal diameter of 2 mm. The sleeve (B) is made from DL-lactide/glycolide copolymer with a molar ratio of 75/25, and the core is made from chitosan with a mean molecular weight of 320 kDa and a deacetylation degree of 82%, wherein the chitosan is in the form of solid fibres (A) with a diameter of 25 µm. The chitosan fibres (A) which constitute the core are placed in the sleeve (B) in a strained state and in parallel to the axis of the said sleeve, and their quantity is 900. In addition, the core is placed in the sleeve (B) so that the sleeve (B) protrudes beyond both core ends. Spaces (C) for the growing nerve fibres are located between the chitosan fibres (A).

In other embodiments, the prosthesis of the invention consists of a sleeve with an internal diameter of 7 mm which contains 11000 chitosan fibres or of a sleeve with an internal diameter of 10 mm which contains 22500 chitosan fibres.

### Example II

Chitosan with Mw = 320 kDa and a deacetylation degree of 82% was used to prepare the fibres. The chitosan was dissolved in 3% acetic acid with addition of glycerine. Chitosan fibres were formed as continuous 300-fibril yarn using an alkaline coagulation bath, water baths, and drying sections, and the yarn was collected on a reel.

The chitosan yarn used for the construction of the prosthesis was characterised by a parallel arrangement of elementary fibres, strength of 14.5 cN/tex, elongation of 12%, crystallinity degree of 38% and ash content of 0.4%.

The solution of DL-lactide/glycolide copolymer with a molar ratio of 75/25 in 1,4-dioxane with a concentration of 3% by weight was poured on a Teflon mould and left at room temperature for 24 hours and subsequently dried at 50°C at 0.07 MPa for 4 days. Subsequently, the film was rinsed several times with demineralised water with glycerine and freeze-dried for 24 hours at -25°C at 0.1 millibar. A transparent film with a thickness of 45 µm was obtained. Subsequently, the film was wound 1.5 times around a bar with diameter corresponding to an internal diameter of the prosthesis sleeve, that is 2 mm, and then subjected to a thermal bonding. In this manner, a sleeve with an internal diameter of 2 mm and a length of 13 mm was obtained. After drying and cooling the place of joint, a bundle of chitosan fibres (core) with a diameter of 2 mm being cut to a predetermined length (7 mm) was inserted into the sleeve. The said bundle consisted of 900 solid fibres, with a diameter of 25 µm each. Such a placement of the core was possible, because the bundle of fibres was sufficiently rigid. The sufficient rigidity is obtained in turn owing to the fibre moulding method during which elementary fibres arranged in parallel manner which hold together during drying are obtained.

In this manner, a prosthesis which consisted of a sleeve in the shape of a cylinder with a wall thickness of 45 µm, an internal diameter of 2 mm and a length of 13 mm as well as of a core with a length of 7 mm and a diameter of 2 mm was obtained. The core was placed in the sleeve so that the sleeve protruded beyond both core ends, whereas the chitosan fibres constituting the core were placed in a strained state and in parallel to the sleeve axis.

The prostheses were irradiated with UV light for 12 hours to ensure sterilisation.

In other embodiments, a sleeve was also prepared according to the aforementioned method with an internal diameter of 7 mm in which 11000 chitosan fibres were placed and a sleeve with an internal diameter of 10 mm in which 22500 chitosan fibres were placed.

### Example III

A transparent film from DL-lactide/glycolide copolymer with a molar ratio of 75/25 was prepared according to Example I. A sleeve with an internal diameter of 2 mm and a length of 22 mm was obtained from the film with a thickness of 45 µm according to Example 1. Subsequently, the prosthesis for regeneration of nerves in the shape of a hollow cylinder was irradiated with UV light for 12 hours to ensure sterilisation.

The prosthesis for regeneration of nerves of the invention may be used in the treatment of nerve injuries in humans, which has been confirmed in pre-clinical *in vivo* animal trials. Immunohistochemistry tests using fluorescence microscopy and confocal microscopy showed growth of regenerating nerve fibres present in the prosthesis core and reconstruction of the missing nerve fragment.

Statistical analysis of the obtained results showed a significant difference between the mean intensity of autotomy in the group in which prostheses with a sleeve from Resomer (PLGA) film which contained a core consisting of chitosan fibres arranged in parallel were used, and the mean intensity of autotomy in the group in which the sleeve with normal saline only was used.

The mean intensity of autotomy in the group with a sleeve from Resomer (PLGA) film filled with normal saline only was higher than in the group where the sleeve contained a core consisting of chitosan fibres arranged in parallel.

The aim of the investigation of the autotomy intensity in animals after the surgery was to correlate the results of morphology analysis of nerve fibres growing through various types of prostheses with animal tests with respect to the sensing level of neuropathic pain resulting from the abnormal regeneration of nerve fibres. Investigations have shown that the prosthesis of the invention provides reconstruction of the missing nerve and radical reduction of intensity of the neuropathic pain.

## Claims

1. A prosthesis for regeneration of nerves consisting of a core with a length of 5-50 mm made from chitosan with a mean molecular weight of 100-500 kDa and a deacetylation degree of 80-98% which is placed in a sleeve in the shape of a cylinder with a wall thickness of 0.04-1.0 mm and an internal diameter of 1-10 mm, the said sleeve being made from DL-lactide/glycolide copolymer with a molar ratio of 75/25, wherein the sleeve protrudes beyond both core ends, **characterised in that** the core is made from chitosan in the form of solid fibres with a diameter of 20-40 µm, wherein the fibres in a strained state are placed in the sleeve in parallel to the axis of the said sleeve, and their quantity is from 150 to 22500.

2. A method of manufacturing the prosthesis for regeneration of nerves **characterised in that** it comprising the following steps:
a) forming a film from DL-lactide/glycolide copolymer by:
- pouring a solution of DL-lactide/glycolide copolymer with a molar ratio of 75/25 in 1,4-dioxane with a concentration of 3% by weight on a Teflon mould;
- leaving the poured solution at room temperature for 24 hours and subsequently drying it at 50°C at 0.07 MPa for 4 days, whereby the film is formed;
- rinsing the formed film several times with demineralised water with glycerine;
- freeze-drying the film for 24 hours at -25°C at 0.1 millibar;
b) forming a sleeve in the shape of a cylinder with a wall thickness of 0.04-1.0 mm and an internal diameter of 1-10 mm from the film obtained in the step a);
c) forming a prosthesis core by placing into the sleeve obtained in the step b) between 150 and 22500 solid chitosan fibres with a mean molecular weight of 100-500 kDa, a deacetylation degree of 80-98% and a diameter of 20-40 µm, wherein the fibres are placed in a strained state and in parallel to the sleeve axis, and the formed core has a length of 5-50 mm and the sleeve protrudes beyond both core ends.

## Patentansprüche

1. Eine Prothese zur Regeneration von Nerven bestehend aus einem Kern mit einer Länge von 5-50mm, hergestellt aus Chitosan mit einem mittleren Molekulargewicht von 100-500 kDa und einem Deacetylierungsgrad von 80-90%, der in einem Schlauch, der die Form eines Zylinders hat mit einer Wanddicke von 0,04-1,0 mm und mit einem Innendurchmesser von 1-10 mm, eingebracht wird, der genannte Schlauch ist hergestellt aus einem DL-Laktid/Glykolid-Kopolymer mit einem Molverhältnis von 75/25, wobei der Schlauch über beide Enden des Kerns herausragt, **dadurch gekennzeichnet, dass** der Kern aus Chitosan in der Form von festen Fasern mit einem Durchmesser von 20-40 µm hergestellt ist, wobei die Fasern in einen beanspruchten Zustand in den Schlauch parallel zur Axe des genannten Schaluchs eingebracht werden, und ihre Anzahl beträgt von 150 bis 22500.

2. Verfahern zur Herstellung einer Prothese zur Regeneration von Nerven, **dadurch gekennzeichnet, dass** es die folgenden Schritte umfasst:
a) Formen eines Films aus einem DL-Laktid/Glykolid-Kopolymer mit einem Molverhältnis von 75/25 durch:
- Gießen einer Lösung aus einem DL-Laktid/Glykolid-Kopolymer mit einem Molverhältnis von 75/25 in 1,4-Dioxan in einer Konzentration von 3 Gew.% auf eine Form aus Teflon;
- Stehenlassen der gegossen Lösung bei Raumtemperatur für 24 Stunden und anschließendes Trocknen bei 50°C bei 0.07 MPa für 4 Tage, wobei ein Film geformt wird,
- Waschen des geformten Films mehrmals mit deminaralisiertem Wasser mit Glyzerin;
- Gefriertrocknen des Films für 24 Stunden bei -25°C bei 0,1 Millibar;
b) Formen des Schlauches in der Form eines Zylinders mit einer Wanddicke von 0,04-1,0 mm und mit einem Innendurchmesser von 1-10 mm aus dem Film, der im Schritt a) erhalten wurde;
c) Formen des Kerns einer Prothese durch das Einbringen in den im Schritt b) erhaltenen Schlauch von zwischen 150 und 22500 festen Chitosan-Fasern, mit einem mittleren Molekulargewicht von 100-500 kDa, mit einem Deacetylierungsgrad von 80-90% und mit einem Durchmesser von 20-40 µm, wobei die Fasern in einen beanspruchten Zustand und parallel zur Achse des Schlauches eingebracht werden, und der geformte Kern hat eine Länge von 5-50 mm und der Schlauch ragt über beide Enden des Kerns heraus.

## Revendications

1. Une prothèse pour la régénération des nerfs constituée d'un noyau d'une longueur de 5 à 50 mm, fabriqué à partir de chitosane de poids moléculaire moyen de 100 à 500 kDa et d'un degré de désacétylation de 80 à 98%, qui est placé dans un manchon en forme de cylindre ayant une épaisseur de paroi de 0,04 à 1,0 mm et un diamètre interne de 1 à 10 mm, ledit manchon étant fabriqué à partir de copolymère de DL-lactide/glycolide ayant un rapport molaire de 75/25 ; le manchon dépasse au-delà de toutes les deux extrémités du noyau, **caractérisée en ce que** le noyau est fabriqué à partir de chitosane sous forme de fibres solides d'un diamètre de 20 à 40 µm ; les fibres à l'état tendu sont placées dans le manchon parallèlement à l'axe dudit manchon, et leur quantité est de 150 à 22500.

2. Un procédé de fabrication de la prothèse pour la régénération de nerfs **caractérisé en ce qu'**il comprend les étapes suivantes:
a) formation d'un film à partir de copolymère DL-lactide/glycolide par:
- verser une solution de copolymère DL-lactide/glycolide ayant un rapport molaire de 75/25 dans du 1,4-dioxane à une concentration de 3% en poids sur un moule en téflon;
- laisser la solution versée à la température ambiante pendant 24 heures et ensuite la sécher à 50° C à 0,07 MPa pendant 4 jours, moyennant quoi le film est formé;
- rincer plusieurs fois le film formé à l'eau déminéralisée avec de la glycérine;
- lyophiliser le film pendant 24 heures à -25° C à 0,1 millibar;
b) formation d'un manchon sous la forme d'un cylindre ayant une épaisseur de paroi de 0,04 à 1,0 mm et un diamètre interne de 1 à 10 mm à partir du film obtenu à l'étape a);
c) formation d'un noyau de prothèse en plaçant dans le manchon obtenu à l'étape b) entre 150 et 22500 fibres solides de chitosane ayant un poids moléculaire moyen de 100-500 kDa, un degré de désacétylation de 80-98% et un diamètre de 20-40 µm ; les fibres sont placeés dans un état tendu et parallèlement à l'axe du manchon, et le noyau formé a une longueur de 5 à 50 mm et le manchon dépasse au-delà de toutes les deux extrémités du noyau.
